# EUROPEAN PATENT APPLICATION

(11) **EP 3 822 255 A1**
(43) Date of publication of application: **19.05.2021**
(21) Application number: 20738481.9
(22) Date of filing: 03.01.2020
(51) Int. Cl.: C07C 271/02, C07K 5/10, C07K 1/04, A61K 31/27, A61P 25/00, A61K 38/08

(54) **SERINE DERIVATIVE COMPOUND FOR PREVENTING OR TREATING CENTRAL NERVOUS SYSTEM DISEASES**

(30) Priority: 07.01.2019 KR 20190001720
(71) Applicant: Astrogen Co., Ltd., Buk-gu Daegu 41566 (KR)
(72) Inventor: KIM, Young Ho, Gunwi-gun Gyeongsangbuk-do 39034 (KR); WHANG, Su-Kyeong, Daegu 41950 (KR); JUN, Do Youn, Gunwi-gun Gyeongsangbuk-do 39034 (KR); JO, Young Kyoung, Daegu 42637 (KR); LEE, Min Yong, Gumi-si Gyeongsangbuk-do 39112 (KR)
(74) Representative: Reitstötter Kinzebach
(86) International application number: PCT/KR2020/000154
(87) International publication number: WO 2020/145584

(57) **Abstract**

The present invention relates to a serine derivative compound having improved blood-brain barrier (BBB) permeability and the use thereof, and more particularly, to a novel serine derivative compound having improved blood-brain barrier permeability compared to L-serine, and a pharmaceutical composition for preventing, treating or alleviating nervous system diseases such as cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis, the pharmaceutical composition containing the compound as an active ingredient. The compound of Formula (I) or a pharmaceutically acceptable salt thereof as an active ingredient for the pharmaceutical composition for preventing or treating central nervous system diseases according to the present invention exhibits significantly improved blood-brain barrier permeability, activates neuronal cell proliferation, and exhibits a neuronal protective effect of inhibiting neuronal cell death resulting from mitochondrial membrane potential damage and/or endoplasmic reticulum stress caused by oxidative stress, and exhibits improved blood-brain barrier permeability. Therefore, the compound has an excellent effect on the prevention, treatment and alleviation of central nervous system diseases such as cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis, and thus is a highly useful invention in the pharmaceutical industry, the food industry and the livestock industry.

## Description

### Technical Field

The present invention relates to a serine derivative compound having improved blood-brain barrier (BBB) permeability and the use thereof, and more particularly, to a novel serine derivative compound having improved blood-brain barrier permeability compared to L-serine, and a pharmaceutical composition for preventing, treating or alleviating nervous system diseases such as cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis, the pharmaceutical composition containing the compound as an active ingredient.

### Background Art

L-serine is not only a member of the protein family *in vivo,* but also an amino acid that plays an important role in biosynthesis of purine, pyrimidine, glycine, cysteine and the like and biosynthesis of sphingomyeline, cerebrosides, D-serine and the like in the brain. Although L-serine may be supplied through food, it can be synthesized *in vivo,* and thus is classified as a non-essential amino acid. However, it is known that L-serine has low ability to pass through the blood-brain barrier, and thus deficient biosynthesis of L-serine in the brain causes neurodevelopmental disorders such as microencephalia, epilepsy and intellectual disability. Thus, L-serine has recently been recognized as a "conditionally essential amino acid" [Smith QR, 1987; de Koning et al., 2003; Metcalf et al., 2018].

It has been reported that reasons why defects of the L-serine biosynthesis pathway in the brain cause neurodevelopmental disorders are that L-serine acts as a neurotrophic factor in neurons [Furuya et al., 2000], and that L-serine is involved in the supply of D-serine, a co-agonist of N-methyl-D-aspartate (NMDA) receptor, and plays an important role in brain neurodevelopment, synapse refinement, neuronal plasticity, and excitotoxicity [Wolosker et al., 2002; Lench et al., 2014].

It has been reported that congenital intellectual disabilities, limb malformation, microcephaly, and neural tube defects are found in patients having serine metabolism defects caused by mutations of 3-phosphoglycerate dehydrogenase (PHGDH), phosphoserine aminotransferase (PSAT) and phosphoserine phosphatase (PSP), which are serine biosynthesis genes, and when L-serine is administered orally to these patients at a dose of 100 to 600 mg/kg/day, symptom alleviation and treatment are possible [Pineda et al., 2000; De Koning et al., 2002]. In addition, it has been reported that co-administration of 200 to 700 mg/kg of serine and 200 to 300 mg/kg of glycerin reduces spasms, increases brain white matter volume, and restores myelination [Tabatabaie et al., 2011; El-Hattab 2016]. Based on these study results, it has become known that supplying L-serine, which is required due to serine metabolism defect in the brain, is very important for the treatment of target diseases.

In addition, since the effect of L-serine was reported in a study on the treatment of Guamanian amyotrophic lateral sclerosis (ALS)/Parkinsonism dementia complex (PDC) by β-N-methylamino-L-alanine (L-BMAA) [Dunlop et al., 2018] and in a clinical study on the treatment of amyotrophic lateral sclerosis [Dunlop et al., 2018], the possibility of L-serine as a therapeutic agent for neurological diseases has become largely known.

The reason why L-serine has therapeutic potential as described above is that serine provides one-carbon necessary for biosynthesis of thymidine and purine in a folate cycle, maintains redox homeostasis *in vivo,* and is also used as a precursor of glycine and cysteine and as an antioxidant precursor of glutathione which is a cellular antioxidant.

Meanwhile, it has been reported that neuronal cell death caused by oxidative stress resulting from damage to mitochondria, which are closely related to bioenergy metabolism and redox maintenance, causes nervous system diseases such as cognitive disorders including autism spectrum disorder (ASD) [Valenti et al., 2014], Alzheimer's disease [Wang et al., 2014], Parkinson's disease [Franco-Iborra et al., 2018], and amyotrophic lateral sclerosis (ALS) [Cozzolino and Carri, 2012]. Mitochondria are organs that perform cell apoptosis or necrosis, and protect cells or repair or remove damaged cells by performing quantitative regulation of the number of mitochondria, such as mitochondrial division and fusion, to maintain cell homeostasis from external environmental stress [Youle and van der Bliek, 2012; Ni et al., 2014].

In particular, neurons are post-mitotic non-proliferating cells that require higher energy metabolism than cells of other tissues, have a high ratio of fatty acids and metal ions that are susceptible to peroxidation, and have a relatively low level of cellular antioxidants, and these neurons are very vulnerable to oxidative stress caused by reactive oxygen species or active nitrogen species [Ogawa et al., 2007; Bhat et al., 2015]. It has been reported that, due to these features of neurons, a common symptom in neurodevelopmental disorders such as cognitive disorder, intellectual disability, microcephaly, epilepsy, autism spectrum disorder, Down's syndrome, Rett's syndrome, and fragile X syndrome, and degenerative neurological diseases such as dementia, Alzheimer's disease, Parkinson's disease, Huntington's disease and amyotrophic lateral sclerosis, is mitochondrial dysfunction caused by oxidative stress, even though the main causes of these diseases differ from each other.

### [Prior Art Documents]

### [Non-Patent Documents]

Bhat AH, Dar KB, Anees S, Zargar MA, Masood A, Sofi MA, Ganie SA. Oxidative stress, mitochondrial dysfunction and neurodegenerative diseases; a mechanistic insight. Biomed Pharmacother. 2015;74:101-110.

Cozzolino M, Carrì MT. Mitochondrial dysfunction in ALS. Prog Neurobiol. 2012;97(2):54-66.

De Koning TJ, Duran M, Van Maldergem L, Pineda M, Dorland L, Gooskens R, Jaeken J, Poll-The BT. Congenital microcephaly and seizures due to 3-phosphoglycerate dehydrogenase deficiency: outcome of treatment with amino acids. J Inherit Metab Dis. 2002;25(2): 119-125.

De Koning TJ, Snell K, Duran M, Berger R, Poll-The BT, Surtees R. L-serine in disease and development. Biochem J. 2003;371(Pt 3):653-661.

Doyle KM, Kennedy D, Gorman AM, Gupta S, Healy SJ, Samali A. Unfolded proteins and endoplasmic reticulum stress in neurodegenerative disorders. J CellMolMed. 2011;15(10):2025-2039.

Dranka BP, Hill BG, Darley-Usmar VM. Mitochondrial reserve capacity in endothelial cells: The impact of nitric oxide and reactive oxygen species. Free Radic Biol Med. 2010;48(7):905-914.

Dunlop RA, Powell JT, Metcalf JS, Guillemin GJ, Cox PA. L-Serine-mediated neuroprotection includes the upregulation of the ER stress chaperone protein disulfide isomerase (PDI). Neurotox Res. 2018;33(1):113-122.

El-Hattab AW. Serine biosynthesis and transport defects. Mol Genet Metab. 2016;118(3):153-159.

Franco-Iborra S, Vila M, Perier C. Mitochondrial quality control in neurodegenerative diseases: Focus on Parkinson's disease and Huntington's disease. Front Neurosci. 2018;12:342.

Furuya S, Tabata T, Mitoma J, Yamada K, Yamasaki M, Makino A, Yamamoto T, Watanabe M, Kano M, Hirabayashi Y. L-serine and glycine serve as major astroglia-derived trophic factors for cerebellar Purkinje neurons. Proc Natl Acad Sci USA. 2000;97(21):11528-11533.

Lench AM, Massey PV, Pollegioni L, Woodhall GL, Jones RS. Astroglial d-serine is the endogenous co-agonist at the presynaptic NMDA receptor in rat entorhinal cortex. Neuropharmacology. 2014;83:118-127.

Metcalf JS, Dunlop RA, Powell JT, Banack SA, Cox PA. L-Serine: a Naturally-occurring amino acid with therapeutic potential. Neurotox Res. 2018;33(1):213-221.

Ni HM, Williams JA, Ding WX. Mitochondrial dynamics and mitochondrial quality control. Redox Biol. 2015;4:6-13.

Ogawa S, Kitao Y, Hori O. Ischemia-induced neuronal cell death and stress response. Antioxid Redox Signal. 2007;9(5):573-587.

Pineda M, Vilaseca MA, Artuch R, Santos S, Garcia Gonzalez MM, Aracil A, Van Schaftingen E, Jaeken J. 3-phosphoglycerate dehydrogenase deficiency in a patient with West syndrome. Dev Med Child Neurol. 2000;42(9):629-633.

Smith QR. Transport of glutamate and other amino acids at the blood-brain barrier. J Nutr. 2000;130(4S Suppl):1016S-1022S.

Tabatabaie L, Klomp LW, Rubio-Gozalbo ME, Spaapen LJ, Haagen AA, Dorland L, De Koning TJ. Expanding the clinical spectrum of 3-phosphoglycerate dehydrogenase deficiency. J Inherit Metab Dis. 2011;34(1):181-184.

Valenti D, de Bari L, De Filippis B, Henrion-Caude A, Vacca RA. Mitochondrial dysfunction as a central actor in intellectual disability-related diseases: an overview of Down syndrome, autism, Fragile X and Rett syndrome. Neurosci Biobehav Rev. 2014;46 Pt2:202-217.

Wang X, Wang W, Li L, Perry G, Lee HG, Zhu X. Oxidative stress and mitochondrial dysfunction in Alzheimer's disease. Biochim Biophys Acta. 2014;1842(8):1240-1247.

Wolosker H, Panizzutti R, De Miranda J. Neurobiology through the looking-glass: D-serine as a new glial-derived transmitter. Neurochem Int. 2002;41(5):327-332.

Youle RJ, van der Bliek AM. Mitochondrial fission, fusion, and stress. Science. 2012;337(6098):1062-1065.

### DISCLOSURE

### Technical Problem

The present invention can be discussed on the extension of the above-described conventional art, and a problem to be solved by the present invention is to provide a novel serine derivative compound having significantly improved blood-brain barrier permeability, and a pharmaceutical composition for preventing or treating central nervous system diseases caused by mitochondrial dysfunction, such as cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis, the pharmaceutical composition containing the compound as an active ingredient.

### Technical Solution

To solve the above-described problem, the present invention provides a compound of the following Formula (I) or a pharmaceutically acceptable salt, solvate, hydrate or isomer of the compound:

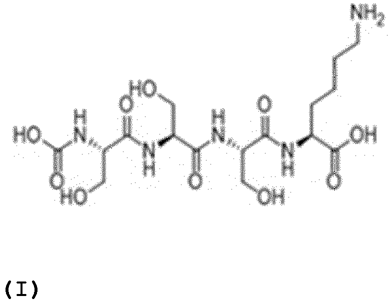

The present invention also provides a method for producing the compound of Formula (I), the method comprising a step of introducing H-Ser(Trt)-OH three times continuously to 2-chlorotrityl chloride resin, introducing H-Lys(Boc)-OH to the resin, and then cleaving the resin.

The present invention also provides a pharmaceutical composition for preventing or treating central nervous system diseases, the pharmaceutical composition containing the compound of Formula (I) or a pharmaceutically acceptable salt thereof as an active ingredient.

The central nervous system diseases are preferably selected from the group consisting of cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis.

The present invention also provides a health functional food for preventing or treating central nervous system diseases, the health functional food containing the compound of Formula (I) or a pharmaceutically acceptable salt thereof as an active ingredient.

The central nervous system diseases are preferably selected from the group consisting of cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis.

The present invention also provides a feed additive composition containing the compound of Formula (I) or a pharmaceutically acceptable salt thereof.

The present invention also provides a reagent composition for inhibiting neuronal cell death, the reagent composition containing the compound of Formula (I) or a pharmaceutically acceptable salt thereof.

The present invention also provides a method for inhibiting neuronal cell death, the method comprising treating neuronal cells with the compound of Formula (I) or a pharmaceutically acceptable salt thereof *in vitro.*

### Advantageous Effects

According to the embodiments, the compound of Formula (I) or a pharmaceutically acceptable salt thereof as an active ingredient for the pharmaceutical composition for preventing or treating central nervous system diseases according to the present invention exhibits significantly improved blood-brain barrier permeability, activates neuronal cell proliferation, and exhibits a neuronal protective effect of inhibiting neuronal cell death resulting from mitochondrial membrane potential damage and/or endoplasmic reticulum stress caused by oxidative stress, and exhibits improved blood-brain barrier permeability. Therefore, the compound has an excellent effect on the prevention, treatment and alleviation of central nervous system diseases such as cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis, and thus is a highly useful invention in the pharmaceutical industry, the food industry and the livestock industry.

### Brief Description of Drawings

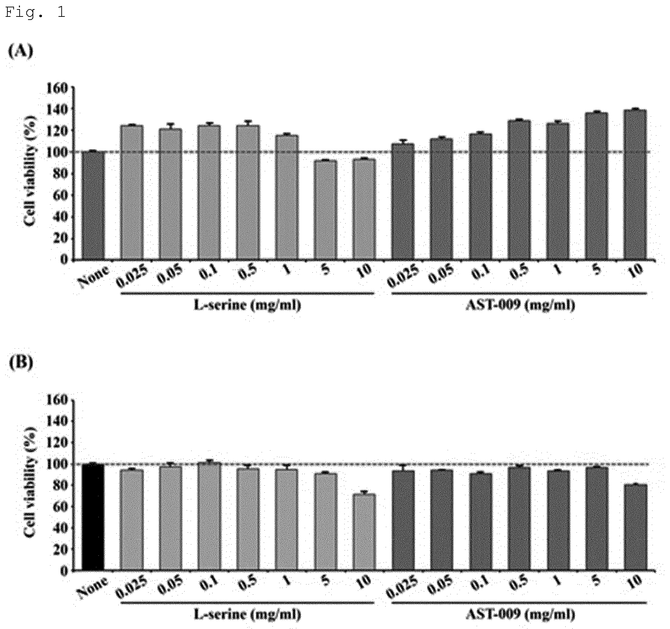

FIG. 1 shows the cell viability of mouse hippocampal neuronal HT-22 cells as a function of the treatment concentration of a serine derivative compound (hereinafter also referred as "AST-009") represented by Formula (I) according to the present invention. (A) shows cell viability in serine/glycerin-deficient medium, and (B) shows cell viability in complete medium.

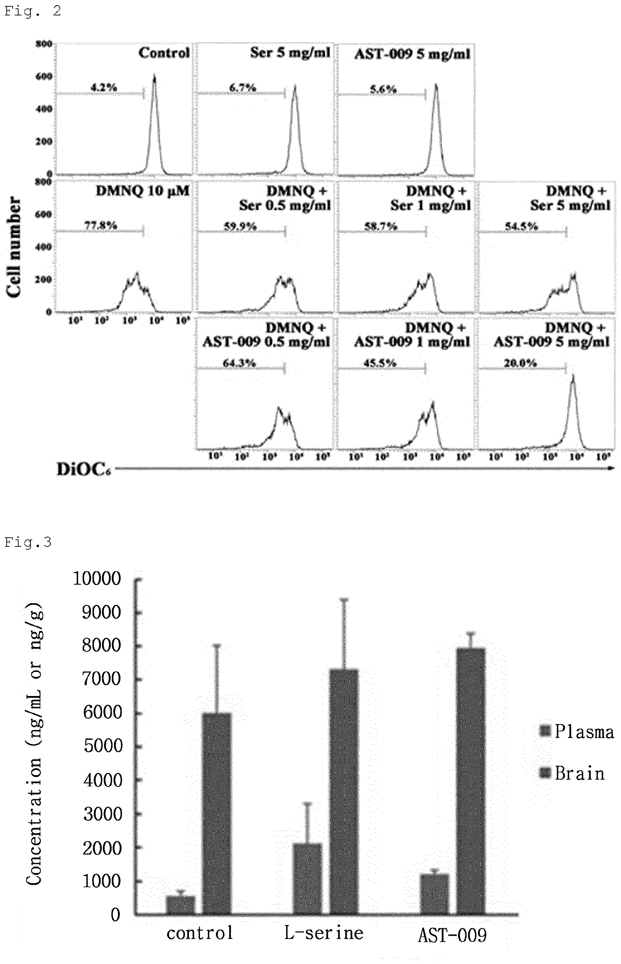

FIG. 2 shows the cell protective effect of the serine derivative compound (AST-009) represented by Formula (I) according to the present invention on the protection of mouse hippocampal neuronal HT-22 cells, treated with DMNQ (2,3-dimethoxy-1,4-napthoquinone), as a function of the treatment concentration of the serine derivative compound.
FIG. 3 shows the blood-brain barrier permeabilities of drugs, determined by administering the serine derivative compound (AST-009) represented by Formula (I) according to the present invention to ICR mice, quantifying the concentrations of the serine derivative compound distributed in the blood and brain, and calculating the ratio of the concentration of the serine derivative compound in the brain to that in the blood.

### Best Mode

Hereinafter, the present invention will be described in detail.

The present inventors have conducted studies to develop a novel serine derivative compound which can improve the blood-brain barrier permeability of L-serine known to exhibit an effect on patients having neurodevelopmental disorders, and which can exhibit effects against diseases related to the central nervous system, such as congenital neurological diseases and degenerative neurological diseases.

Therefore, the present invention provides a compound of the following Formula (I) or a pharmaceutically acceptable salt, solvate, hydrate or isomer of the compound:

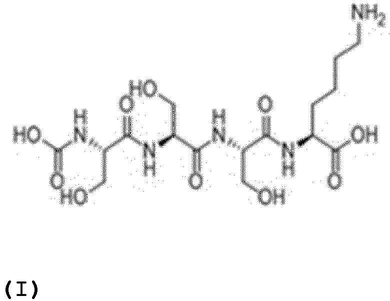

The compound of Formula (I) may exist as a base-addition salt or an acid-addition salt. The addition salt is included as a part of the present invention. Although the salt is advantageously prepared with a pharmaceutically acceptable acid, for example, salts of other acids useful for purifying or isolating the compound of Formula (I) are also included as a part of the present invention. The acids may be, for example, picric acid, oxalic acid or optically active acids such as tartaric acid, dibenzoyl tartaric acid, mandelic acid or a camphorsulfonic acid, and acids which form physiologically acceptable salts such as hydrochloride, hydrobromide, sulfate, hydrogen sulfate, dihydrogen phosphate, maleate, fumarate, 2-naphthalene sulfonate or para-toluenesulfonate. For physiologically acceptable salts, reference may be made to Handbook of Pharmaceutical Salts: Properties, Selection and Use by Stahl and Wermuth (Wiley-VCH, 2002)

The solvates or hydrates may be obtained directly from the synthetic process, and the compound (I) may be isolated in the form of a hydrate, for example a mono- or hemi-hydrate or a solvate of a reaction or purification solvent.

In addition, the compound of formula (I) may exist in the form of isomers, for example, enantiomers, diastereomers and rotational isomers. Enantiomers, diastereomers and rotational isomers of the compound of formula (I) are included as a part of the present invention.

The compound of Formula (I) according to the present invention may be synthesized with high yield and purity by a production method as described below.

Therefore, the present invention provides a method for producing the compound of Formula (I), the method comprising a step of introducing H-Ser(Trt)-OH three times continuously to 2-chlorotrityl chloride resin, introducing H-Lys(Boc)-OH to the resin, and then cleaving the resin.

A specific method for producing the serine derivative compound of Formula (I) according to the present invention will now be described with reference to the following Scheme.

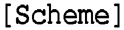

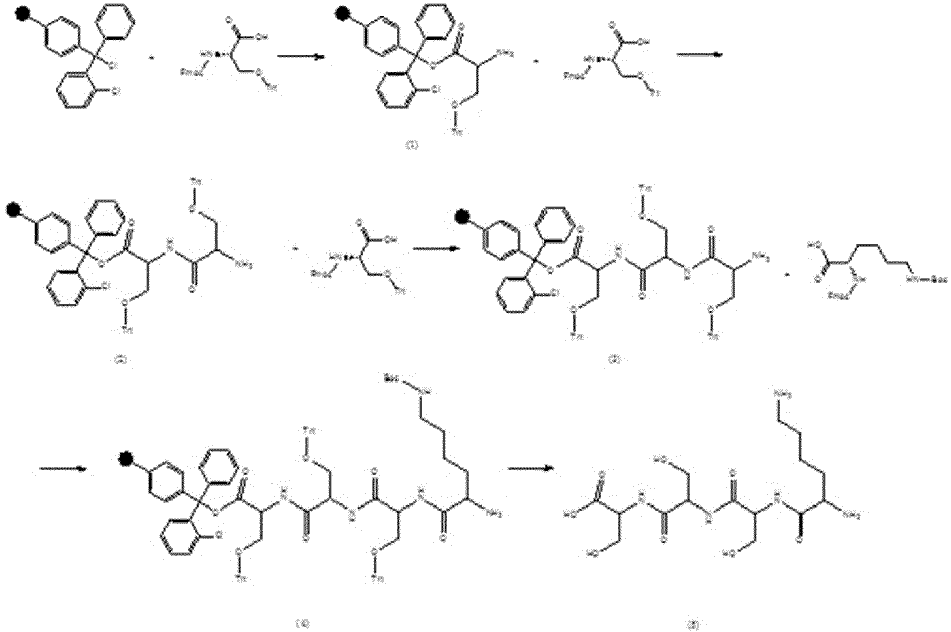

### (1) Swelling of CLTR and Introduction of H-Ser(Trt)-OH

1) 2-Chlorotrityl chloride resin (0.1 mmol) and MC (0.4 L) are placed in a 4-L reactor and swollen for 2 hours.
2) Fmoc-Ser(trt)-OH (1.5 eq.) and DIPEA (3 eq.) in DMF (0.2 L) are added to the swollen resin and allowed to react at room temperature for 6 hours.
3) After completion of the reaction, the resin is washed with DMF (0.2 L).
4) A mixture of MC/MeOH/DIPEA (255:30:15) is added to the washed resin, and a capping reaction with shaking is performed at room temperature for 2 hours.
5) After completion of the capping, the resin is washed with DMF (0.2 L).
6) A de-blocking solution (20% piperidine in 0.3 L of DMF) is added to the resin, followed by reaction at room temperature for 2 hours.
7) After completion of the de-blocking, the resin is washed three times with DMF (0.2 L) and washed with twice with MC (0.2 L).

### (2) Introduction of H-Ser (Trt) -OH

1) After completion of process (1) above, a solution of Fmoc-Ser(trt)-OH (1.5 eq.) and HOBt (1.5 eq.) in DMF (0.3 L) is added to the reactor, and then DIPEA (2 eq.) is added quickly to a solution of HBTU (1.5 eq.) in DMF (0.2 L) and added to the reactor, followed by reaction at room temperature for 6 hours or more.
2) After completion of the reaction, the resin is washed with DMF (0.2 L).
3) A de-blocking solution (20% piperidine in 0.3 L of DMF) is added to the resin, followed by reaction at room temperature for 2 hours.
4) The resin is washed three times with DMF (0.2 L) and washed twice with MC (0.2 L).

### (3) Introduction of H-Ser (Trt) -OH

1) After completion of process (2) above, a solution of Fmoc-Ser(trt)-OH (1.5 eq.) and HOBt (1.5 eq.) in DMF (0.3 L) is added to the reactor, and then DIPEA (2 eq.) is added quickly to a solution of HBTU (1.5 eq.) in DMF (0.2 L) and added to the reactor, followed by reaction at room temperature for 6 hours or more.
2) After completion of the reaction, the resin is washed with DMF (0.2 L).
3) A de-blocking solution (20% piperidine in 0.3 L of DMF) is added to the resin, followed by reaction at room temperature for 2 hours.
4) The resin is washed three times with DMF (0.2 L) and washed twice with MC (0.2 L).

### (4) Introduction of H-Lys (Boc) -OH

1) After completion of process (3) above, a solution of Fmoc-Lys(Boc)-OH (1.5 eq.) and HOBt (1.5 eq.) in DMF (0.3 L) is added to the reactor, and then DIPEA (2 eq.) is added quickly to a solution of HBTU (1.5 eq.) in DMF (0.2 L) and added to the reactor, followed by reaction at room temperature for 6 hours or more.
2) After completion of the reaction, the resin is washed with DMF (0.2 L).
3) A de-blocking solution (20% piperidine in 0.3 L of DMF) is added to the resin, followed by reaction at room temperature for 2 hours.
4) The resin is washed three times with DMF (0.2 L) and washed twice with MC (0.2 L).

### (5) Synthesis of Cleaved Crude H-Lys-Ser-Ser-Ser-OH

1) After completion of the synthesis, the resin is cleaved by adding TFA, TIS and H₂O thereto.
2) The cleaved resin is filtered through a glass filter, and the filtrate is dispersed in ether and then centrifuged to obtain a crude product.

### (6) Purification of Final Product (H-Lys-Ser-Ser-Ser-OH)

1) The dry crude product is dissolved in water and purified by Prep LC to obtain 90% or more pure H-Lys-Ser-Ser-Ser-OH.
2) The purified product is freeze-dried to obtain H-Lys-Ser-Ser-Ser-OH as a final product.

The compound of Formula (I) or pharmaceutically acceptable salt thereof according to the present invention may improve the blood-brain barrier permeability of L-serine, may exhibit effects against diseases related to the central nervous system, such as congenital neurological diseases and degenerative neurological diseases, and thus may be used as a pharmaceutical drug for preventing or treating these diseases.

Therefore, the present invention provides a pharmaceutical composition for preventing or treating central nervous system diseases, the pharmaceutical composition containing the compound of Formula (I) or a pharmaceutically acceptable salt thereof as an active ingredient.

The active ingredient of the present invention may be applied as a pharmaceutical drug against central nervous system diseases, wherein the central nervous system diseases are preferably selected from the group consisting of cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis.

The active ingredient preferably induces activation of neuronal cell proliferation. The term "activation of neuronal cell proliferation" may be understood to include both an action of promoting neuronal cell division and an action of inhibiting neuronal apoptosis or necrosis.

The active ingredient preferably has neuronal cell protection activity. The term "neuronal cell protection" refers to an action of inhibiting neuronal apoptosis or necrosis from being caused by external factors or cellular internal factors.

The neuronal cell protection is preferably protection from oxidative stress. The term "oxidative stress" means that cells are in an abnormal state due to reactive oxygen species.

The protection from oxidative stress is preferably achieved by inhibition of cell death caused by mitochondrial membrane potential damage.

The protection from oxidative stress is preferably achieved by inhibition of cell death caused by endoplasmic reticulum stress.

The active ingredient preferably has the ability to permeate the blood-brain barrier. The active ingredient of the present invention significantly improves the blood-brain barrier permeability of L-serine, and can be effectively delivered into the brain when administered to a patient with L-serine biosynthesis defects.

For use, the pharmaceutical composition of the present invention may be formulated in oral dosage forms such as pills, granules, tablets, capsules, suspensions, emulsions, syrups or aerosols, or other various forms such as sterile injectable solutions, depending on the intended use thereof according to conventional methods. The pharmaceutical composition may be administered orally or may be administered through various routes including intravenous, intraperitoneal, subcutaneous, rectal and topical routes.

This pharmaceutical composition may further contain carriers, excipients or diluents, and examples of suitable carriers, excipients or diluents that may be contained in the composition include lactose, dextrose, sucrose, sorbitol, mannitol, xylitol, erythritol, maltitol, starch, acacia gum, alginate, gelatin, calcium phosphate, calcium silicate, cellulose, methyl cellulose, amorphous cellulose, polyvinyl pyrrolidone, water, methylhydroxybenzoate, propylhydroxybenzoate, talc, magnesium stearate, and mineral oil.

In addition, the pharmaceutical composition of the present invention may further contain a filler, an anti-aggregating agent, a lubricant, a wetting agent, a fragrance, an emulsifier, a preservative, and the like.

The pharmaceutical composition of the present invention may be administered in a pharmaceutically effective amount. As used herein, the term "pharmaceutically effective amount" refers to an amount sufficient to treat diseases, at a reasonable benefit/risk ratio applicable to any medical treatment. The effective dosage level of the composition may be determined depending on factors, including the kind and severity of the disease of a patient, the activity of the drug, sensitivity to the drug, the time of administration, the route of administration, excretion rate, the duration of treatment, drugs used in combination with the composition, and other factors known in the medical field.

The pharmaceutical composition of the present invention may be administered individually or in combination with other therapeutic agents, and may be administered sequentially or simultaneously with conventional therapeutic agents. The pharmaceutical composition may be administered in a single or multiple dosage form. It is important to administer the composition in the minimum amount that can exhibit the maximum effect without causing side effects, in view of all the above-described factors, and this amount can be easily determined by a person skilled in the art.

In a preferred embodiment, the effective amount of the active ingredient in the pharmaceutical composition of the present invention may vary depending on the patient's age, sex and bodyweight. In general, the active ingredient may be administered at a dose of 1 to 5,000 mg/kg bodyweight/day, preferably 100 to 3,000 mg/kg bodyweight/day, daily or every other day, or may be administered one to three times a day. However, since the dose may increase or decrease depending on the route of administration, the severity of the disease, the patient's sex, bodyweight and age, etc., the dose is not intended to limit the scope of the present disclosure in any way.

The pharmaceutical composition of the present invention may be administered to a subject through various routes. All modes of administration can be contemplated. For example, the composition may be administered orally, intrarectally, or by intravenous, intramuscular, subcutaneous, intrauterine, intrathecal or intracerebroventricular injection.

In the present invention, "administration" means providing a given substance to a patient by any suitable method. The pharmaceutical composition of the present invention may be administered orally or parenterally through all general routes as long as it can reach the target tissue. In addition, the composition of the present invention may also be administered using any device capable of delivering the active ingredient to target cells.

In the present invention, the term "subject" is not particularly limited, but includes, for example, humans, monkeys, cattle, horses, sheep, pigs, chicken, turkeys, quails, cats, dogs, mice, rats, rabbits or guinea pigs, and preferably refers to mammals, more preferably humans.

The present invention also provides a health functional food for preventing or alleviating central nervous system diseases, the health functional food containing the compound of Formula (I) or a pharmaceutically acceptable salt thereof as an active ingredient.

The central nervous system diseases are preferably selected from the group consisting of cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis.

The health functional food of the present invention may be variously used in foods and beverages effective in preventing and improving diseases related to the central nervous system.

Foods containing the active ingredient of the present invention include various foods, for example, beverages, gums, teas, vitamin complexes, health supplement foods and the like, and may be used in the form of powders, granules, tablets, capsules or beverages.

The active ingredient of the present invention may generally be added in an amount of 0.01 to 15 wt% based on the total food weight. For a health beverage composition, the active ingredient may be added in an amount of 0.02 to 10 g, preferably 0.3 to 1 g, based on 100 ml of the health beverage composition.

The health functional food of the present invention may additionally contain food-acceptable additives, for example, natural carbohydrates and various flavoring agents, in addition to containing the compound as an essential component at the indicated percentage.

Examples of the natural carbohydrates include conventional sugars, such as monosaccharides (e.g., glucose, fructose, etc.), disaccharides (e.g., maltose, sucrose, etc.), polysaccharides (e.g., dextrin, cyclodextrin, etc.), and sugar alcohols such as xylitol, sorbitol, erythritol or the like.

Examples of the flavoring agents that may be used in the present invention include thaumatin, rebaudioside A, glycyrrhizin, saccharin, aspartame, etc. The flavoring agent is used in an amount of about 1 to 20 g, preferably about 5 to 12 g, based on 100 mL of the health functional food of the present invention.

In addition, the health functional food of the present invention may contain various nutrients, vitamins, minerals, flavoring agents such as synthetic flavoring agents and natural flavoring agents, colorants, extenders, pectic acid and its salt, alginic acid and its salt, organic acids, protective colloidal thickeners, pH adjusting agents, stabilizers, preservatives, glycerin, alcohol, carbonizing agents as used in carbonated beverages, etc.

Additionally, the health functional food of the present invention may contain fruit flesh that is used for the preparation of natural fruit juice, fruit juice beverages or vegetable beverages. These components may be used individually or in combination. The content of these additives is generally selected in the range of 0.01 to about 20 parts by weight based on 100 parts by weight of the health functional food.

The present invention also provides a feed additive composition containing the compound of Formula (I) or a pharmaceutically acceptable salt thereof.

The feed additive composition may be for animals. The "animals" refers to a group of organisms corresponding to plants, which consumes organic matter as nutrients and in which digestive, excretory and respiratory organs are differentiated. Specifically, the animals may be echinoderms, crustaceans, mollusks, fish, amphibians, reptiles, birds, or mammals. Preferably, the animals are echinoderms such as sea urchins or sea cucumbers; arthropods including crustaceans such as crab, shrimp, and Chinese white shrimp; mollusks such as cephalopods, gastropods, or bivalve; fish such as red bream, sea bream, cod, or halibut or flatfish; birds including poultry such as pheasant or chicken; or mammals such as pigs, cattle, sheep, horses, goats, dogs, or cats.

The feed additive composition may further contain grains, vegetable protein feed, animal protein feed, sugar or a dairy product, in addition to the active ingredient of the present invention. The grains may specifically be ground or crushed wheat, oats, barley, corn and rice; the vegetable protein feed may specifically be based on rapeseed, soybean and sunflower; the animal protein feed may specifically be blood meal, meat meal, bone meal and fish meal; and the sugar or dairy product may specifically be a dry component consisting of various milk powders and whey powders.

The food additive composition may further contain components such as nutritional supplements, digestion and absorption enhancers, growth promoters or disease preventive agents.

The feed additive composition of the present invention may vary depending on the purpose of use and conditions of use of feed. For example, the feed additive composition may be contained in an amount of 0.1 to 100 g based on 1 kg of finally produced feed.

In addition, the feed additive composition may be prepared into consistent viscous coarse or granular materials according to the degree of pulverization of the components thereof. The composition may be supplied as a mesh or may be formed into a desired separate shape for further processing and packaging, and may be subjected to pelletization, expansion or extrusion processes for storage. For the easiness of storage, an excess amount of water may preferably be removed from the composition by drying.

The present invention also provides a reagent composition for research, preferably a reagent composition for inhibiting neuronal cell death, the reagent composition containing the compound of Formula (I) or a pharmaceutically acceptable salt thereof.

The neuronal cells may be primary neuronal cells, transformed neuronal cells, or neuronal cell lines.

The reagent may be used for activation of neuronal cells, activation of neuronal cell proliferation by increased oxygen consumption rate of mitochondria, protection of neuronal cells, inhibition of neuronal cell damage caused by oxidative stress, inhibition of neuronal cell death resulting from mitochondrial membrane potential damage caused by oxidative stress, or inhibition of neuronal cell death resulting from endoplasmic reticulum stress caused by oxidative stress.

The present invention also provides a method for inhibiting neuronal cell death, the method comprising treating neuronal cells with the reagent of the present invention, which contains the compound of Formula (I) or a pharmaceutically acceptable salt thereof.

According to the above method, it is possible to obtain effects of activation of neuronal cells, activation of neuronal cell proliferation by increased oxygen consumption rate of mitochondria, protection of neuronal cells, inhibition of neuronal cell damage caused by oxidative stress, inhibition of neuronal cell death resulting from mitochondrial membrane potential damage caused by oxidative stress, or inhibition of neuronal cell death resulting from endoplasmic reticulum stress caused by oxidative stress.

In the above method, a method for cell culture method, a method for treatment with the reagent, etc. are matters that are obvious to those of ordinary skill in the art. In particular, the treatment concentration of the reagent, etc., may be appropriately modified within the range of matters described in the present specification or within a range in which the effect of the reagent does not change.

The method is preferably performed *in vitro.*

### Mode for Invention

Hereafter, the present invention will be described in more detail with reference to specific examples. The following examples describe one preferred embodiment of the present invention, and it is to be understood that the scope of the present invention is not limited by the contents described in the following examples.

### [Examples]

### 1. Materials and Method

### 1.1. Medium and Reagents

Cell culture reagents, including Dulbecco's Modified Eagle Medium (DMEM), fetal bovine serum (FBS), 4-(2-hydroxyethyl)piperazine-1-ethanesulfonic acid (HEPES) and streptomycin-penicillin, were purchased from Gibco BRL (Grand Island, USA).

### 1.2. Production of Novel Serine Derivative Compound (AST-009)

2-Chlorotrityl chloride resin (0.1 mmol) and MC (0.4 L) were added to a 4-L reactor and swollen for 2 hours. Fmoc-Ser(trt)-OH (1.5 eq.) and DIPEA (3 eq.) in DMF (0.2 L) were added to the swollen resin and allowed to react at room temperature for 6 hours. After completion of the reaction, the resin was washed with DMF (0.2 L). A mixture of MC/MeOH/DIPEA (255:30:15) was added to the resin, and the resin was subjected to a capping reaction with shaking at room temperature for 2 hours and then washed with DMF (0.2 L). A de-blocking solution (20 % piperidine in DMF=0.3 L) was added to the resin and allowed to react at room temperature for 2 hours. After completion of de-blocking, the resin was washed three times with DMF (0.2 L) and washed twice with MC (0.2 L). Thereafter, a solution of Fmoc-Ser(trt)-OH (1.5 eq.) and HOBt (1.5 eq.) in DMF (0.3 L) was added to the reactor, and then DIPEA (2 eq.) was added quickly to a solution of HBTU (1.5 eq.) in DMF (0.2 L) and added to reactor, followed by reaction at room temperature for 6 hours or more. After completion of the reaction, the resin was washed with DMF (0.2 L), and a de-blocking solution (20% piperidine in DMF=0.3 L) was added to the resin, followed by reaction at room temperature for 2 hours. Then, the resin was washed three times with DMF (0.2 L) and washed twice with MC (0.2 L). Thereafter, a solution of Fmoc-Ser(trt)-OH (1.5 eq.) and HOBt (1.5 eq.) in DMF (0.3 L) was added to the reactor, and then DIPEA (2 eq.) was added quickly to a solution of HBTU (1.5 eq.) in DMF (0.2 L) and added to reactor, followed by reaction for 6 hours or more. Then, the resin was washed with DMF (0.2 L). A de-blocking solution (20% piperidine in DMF=0.3 L) was added to the resin and allowed to react at room temperature for 2 hours. Then, the resin was washed three times with DMF (0.2 L) and twice with MC (0.2 L). Thereafter, a solution of Fmoc-Ser(trt)-OH (1.5 eq.) and HOBt (1.5 eq.) in DMF (0.3 L) was added to the reactor, and then DIPEA (2 eq.) was added quickly to a solution of HBTU (1.5 eq.) in DMF (0.2 L) and added to reactor, followed by reaction for 6 hours or more. Then, the resin was washed with DMF (0.2 L). A de-blocking solution (20% piperidine in DMF=0.3 L) was added to the resin and allowed to react at room temperature for 2 hours, and then the resin washed three times with DMF (0.2 L) and twice with MC (0.2 L). Thereafter, the synthesized resin was cleaved by adding TFA, TIS and H₂O thereto. Then, the cleaved resin was filtered through a glass filter, and the filtrate was dispersed in ether and then centrifuged to obtain a crude product. Finally, the dry crude product was dissolved in water and purified by Prep LC to obtain 90% or more pure H-Lys-Ser-Ser-Ser-OH, and the purified product was freeze-dried to obtain H-Lys-Ser-Ser-Ser-OH as a final product.

### 1.3. Cell Culture

Murine hippocampal neuronal cell line HT-22 was cultured in Dulbecco's Modified Eagle's Medium (DMEM) supplemented with 10% fetal bovine serum (FBS) and 100 µg/ml gentamycin at 37°C under a 5% CO₂ atmosphere. In this Example, cells at passage 15 or below were used.

### 1.4. Cell Viability Assay

Cell proliferation activity was examined by an MTT assay for measuring cell viability. First, hippocampal neuronal cell HT-22 (1×10⁴ cells) was incubated with each of serially diluted sample solutions in 96-well plates for 16 hours, and then mixed with 50 µl of MTT (3-(4,5-dimethyl thiazolyl)2,5-diphenyl tetrazolium bromide) solution (1.1 mg/ml), followed by additional incubation for 4 hours. The formed formazan crystal was dissolved in 150 µl of MTT solution, and the optical density (OD) at 540 nm was measured using a plate reader.

### 1.5. Flow Cytometry Assay

First, 1×10⁶ cells were washed three times with 2% FBS-containing PBS solution, and the washed cells were suspended in 70% ethanol and fixed at 4°C for 1 hour. The fixed cells were washed twice with the same solution, suspended in 250 µl of RNase A solution (50 µg/ml concentration), and incubated at 37°C for 30 minutes to remove RNA from the cells. Then, DNA in the cells was stained at room temperature for 20 minutes by adding 250 µl of 1.12% sodium citrate buffer (pH 8.45) containing 50 µg/ml of propidium iodide. The cells were analyzed with a flow cytometer (FACS Calibur), and the distribution of the cell cycle was examined based on the content of stained DNA in each cell.

### 1.6. Analysis of Blood-Brain Barrier (BBB) Permeability

Each of L-serine and AST-099 was administered to 7-week-old ICR mice (n = 3) at a dose of 500 mg/kg, and then the brain tissue and the blood were collected. The concentrations of L-serine distributed in the blood and the brain were quantified using LC/MS, and the BBB permeability was compared between the drugs by calculating the ratio of the concentration of L-serine in the brain to that in the blood.

### 2. Results

### 2.1. Development of Novel L-Serine Derivative

L-serine is a nonessential amino acid that is biosynthesized *in vivo.* However, L-serine has low BBB permeability, and hence serious neurodevelopmental disorders are caused unless sufficient L-serine is applied to the brain. Since L-serine in blood is selectively transported by neutral amino acid transporter ASC-1 (alanine, serine and cysteine transporters), the supply of L-serine for alleviating and treating neurodevelopmental disorder-related diseases is significantly limited. Thus, in order to develop a serine derivative not only having excellent cell protection activity, like L-serine, but also having improved BBB permeability, 15 different L-serine derivatives were developed. Among the 15 developed derivatives, the serine derivative compound (AST-009) of the present invention was selected through pharmacokinetic evaluation and cytotoxicity evaluation.

### 2.2. Evaluation of Neuronal Cell Proliferation Activity

To evaluate the neuronal cell proliferation activity of the selected serine derivative compound (AST-009) of the present invention, the comparison of cell proliferation activity between AST-009 and L-serine as a control was performed by adding each substance at various concentrations (25 to 10,000 µg). As a result, as shown in FIG. 1, it was confirmed that, in complete medium, there was no significant difference in the effect on cell proliferation between L-serine and AST-009 (FIG. 1B), but in L-serine/glycerin-deficient medium, L-serine and AST-009 all activated HT-22 cell proliferation compared to the untreated group (FIG. 1A). At this time, L-serine activated cell proliferation up to 125% at a treatment concentration of up to 500 µg/ml, but rather slightly inhibited cell proliferation at a concentration of 1 mg/ml or more. On the contrary, it was confirmed that AST-009 activated the proliferation of hippocampal HT-22 cells up to 139% without cytotoxicity even at a treatment concentration of 10,000 µg/ml (=10 mg/ml).

### 2.3. Evaluation of Neuronal Cell Protection Activity

The cell protective activity of the selected drug (AST-009) against DMNQ-induced oxidative stress was compared by DiOC₆ staining. As a result, as shown in FIG. 2, it was confirmed that, when the cells were treated with DMNQ (10 µM) alone, the cell damage rate was 77.8%, whereas, when the cells were treated with L-serine at concentrations of 0.5, 1 and 5 mg/ml, the cell damage rates at these concentrations were 59.9%, 58.7% and 54.5%, respectively, indicating that the cells were protected from membrane potential loss. On the contrary, when the cells were treated with AST-009 at the same concentrations, the cell damage rates at these concentrations were 64.3%, 45.5% and 20.0%, respectively, which were lower than those of the cells treated with L-serine, indicating that the membrane potential loss was lowered. Thus, it could be seen that the cell protective activity of AST-009 was much stronger than that of L-serine. That is, it could be confirmed that AST-009 not only had a better ability to activate cell proliferation than L-serine, but also had an excellent activity of protecting neuronal cells from oxidative stress compared to L-serine.

### 2.4. Analysis of Blood-Brain Barrier (BBB) Permeability of Novel L-Serine Derivative

The delivery of the selected serine derivative AST-009 into the brain was compared with that of L-serine, and the results are shown in FIG. 3 and Table 1 below. As shown in FIG. 3 and Table 1, the C_{brain}/Cₚₗₐₛₘ value of the AST-009 drug was 6.66±1.03, which was higher than that of L-serine (4.16±2.03). Therefore, it was confirmed that AST-009 was a drug having significantly improved BBB permeability compared to L-serine.

**[Table 1]**

| Compounds | Concentration (ng/mL or ng/g) | | C_{brain}/Cₚₗₐₛₘₐ |
|---|---|---|---|
| | Plasma | Brain tissue | |
| Control | 547 ± 161 | 6005 ± 2014 | 12.3 ± 6.8 |
| L-serine | 2121 ± 1192 | 7314 ± 2071 | 4.16 ± 2.03 |
| AST-009 | 1205 ± 120* | 7944 ± 427 | 6.66 ± 1.03 |

| | | | |
|---|---|---|---|
| * *P<0.05 vs Control* | | | |

[National R&D Project That Supported This Invention]
[Grant Number] S2611222
[Ministry Name] Ministry of Small and Medium Business
[Research Management Professional Body] Korea Technology and Information Promotion Agency for Small & Medium Enterprises
[Research Project Name] Start-up Growth-Technology Development Project
[Research Title] Development of functional food for improving autism spectrum disorder
[Contribution Ratio] 1/1
[Managing Department] Astrogen Co., Ltd.
[Research Period] June 29, 2018 through June 28, 2019

## Claims

1. A compound of the following Formula (I) or a pharmaceutically acceptable salt, solvate, hydrate or isomer of the compound:

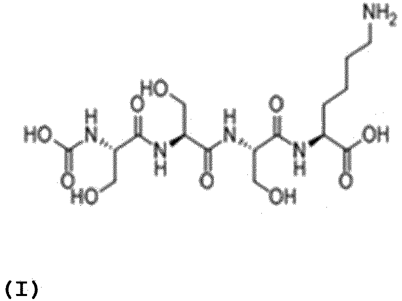

2. A method for producing a compound of the following Formula (I), the method comprising a step of introducing H-Ser(Trt)-OH three times continuously to 2-chlorotrityl chloride resin, introducing H-Lys(Boc)-OH to the resin, and then cleaving the resin:

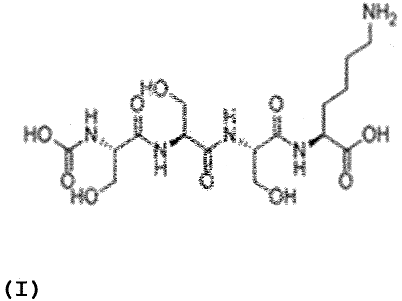

3. A pharmaceutical composition for preventing or treating central nervous system diseases, the pharmaceutical composition containing a compound of the following Formula (I) or a pharmaceutically acceptable salt thereof as an active ingredient:

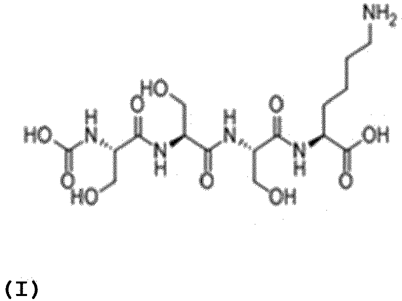

4. The pharmaceutical composition of claim 3, wherein the central nervous system diseases are selected from the group consisting of cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis.

5. A health functional food for preventing or treating central nervous system diseases, the pharmaceutical composition containing a compound of the following Formula (I) or a pharmaceutically acceptable salt thereof as an active ingredient:

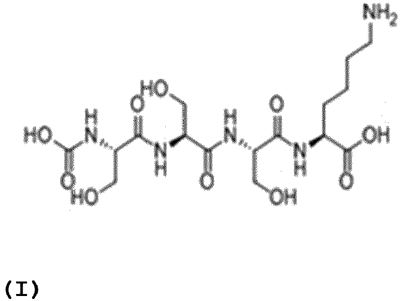

6. The health functional food of claim 5, wherein the central nervous system diseases are selected from the group consisting of cognitive disorder, intellectual disability, microcephaly, epilepsy, neurodevelopmental disorder, dementia, autism spectrum disorder, Down's syndrome, Rett's syndrome, fragile X syndrome, Alzheimer's disease, Parkinson's disease, Huntington's disease, and amyotrophic lateral sclerosis.

7. A food additive composition containing a compound of the following Formula (I) or a pharmaceutically acceptable salt thereof as an active ingredient:

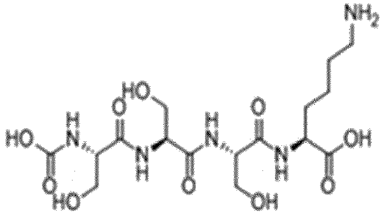

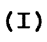

8. A reagent composition for inhibiting neuronal cell death, the reagent composition containing a compound of the following Formula (I) or a pharmaceutically acceptable salt thereof as an active ingredient:

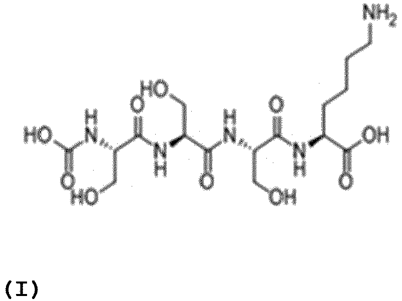

9. A method for inhibiting neuronal cell death, the method comprising treating neuronal cells with a compound of the following Formula (I) or a pharmaceutically acceptable salt thereof *in vitro:*
